# EUROPEAN PATENT APPLICATION

(11) **EP 2 180 319 A1**
(43) Date of publication of application: **28.04.2010**
(21) Application number: 09173609.0
(22) Date of filing: 21.10.2009
(51) Int. Cl.: G01N 33/52, G01N 33/543

(54) **Biological test strip**

(30) Priority: 21.10.2008 TW 97140337
(71) Applicant: Kaiwood Technology Co., Ltd., Tainan County, Hsin-Shi (TW); Lee, Jin Po, Carlsbad, CA 92011 (US)
(72) Inventor: Lee, Jin Po, Carlsbad, CA 92011 (US); Chuang, Tsung-kai, Tainan City 701 (TW)
(74) Representative: Barth, Stephan Manuel

(57) **Abstract**

A biological test strip is disclosed and includes a source end, at least one test line and at least two control lines. The source end is used for dropping a sample liquid thereon. The at least one test line is used to react with at least one sample of the sample liquid to generate a colorization reaction. The at least two control lines, are used to react with the sample liquid to generate at least two colorization reactions with different color darkness, so as to define a predetermined maximum concentration value and a predetermined minimum concentration value, both of which can be used as color comparison references for the at least one test line.

## Description

### FIELD OF THE INVENTION

The present invention relates to a biological test strip, and more particularly to a biological test strip having at least two control lines for increasing the detection accuracy.

### BACKGROUND OF THE INVENTION

Recently, various biological detection experiments are developed for satisfying needs of hospitals, clinics, researchers, or personal users. For this purpose, manufacturers continuously improve and develop various simple biological test strips which can speedily show the detection result after being used, in order to increase the operational convenience. According to the difference between biological specimens, there are various different design principles of the biological test strips. Furthermore, the biological test strips can be used to detect samples (such as ethanol, medicines, cancer molecules or other related disease molecules) in the urine or blood, while some biological test strips can be used to detection the pregnancy.

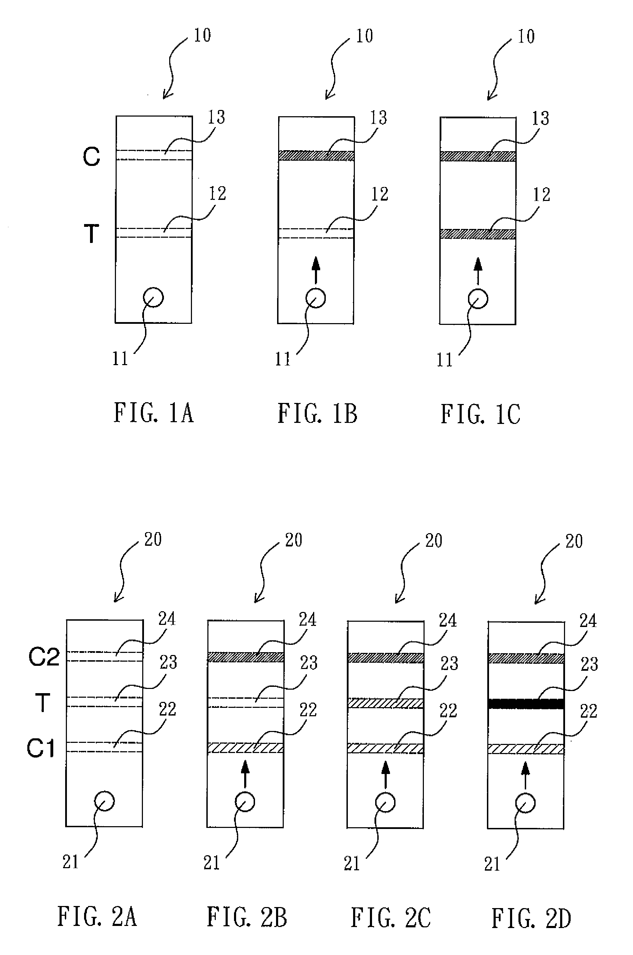

For example, referring now to Fig. 1A, a traditionally biological test strip is illustrated. As shown, the biological test strip comprises a substrate 10 which is generally a paper strip and provided with a source end 11, a test line 12 (also labeled by "T"), and a control line 13 (also labeled by "C"), wherein the paper strip is designed based on immuno-chromatographic principle. The source end 11 is used for dropping a sample liquid thereon. If the sample liquid is dropped, the sample liquid and a sample therein will extend toward the test line 12 and the control line 13. The test line 12 is used to react with the sample of the sample liquid to determine if the sample exists in the sample liquid. The control line 13 are used to react with the sample liquid to be used as a color comparison reference for determining if the sample liquid has extended and passed through the test line 12 and if the detection operation is finished.

Referring to Fig. 1A, before the sample liquid is dropped, the test line 12 and the control line 13 are colorless. Referring now to Fig. 1B, after the sample liquid is dropped for a test, the test line 12 is still colorless, but the control line 13 generates a colorization reaction. In this case, the test result is defined as a negative reaction which means that the concentration of the sample (such as ethanol or medicines) in the sample liquid is zero or less than a predetermined minimum concentration value. Alternatively, referring now to Fig. 1C, after the sample liquid is dropped for a test, each of the test line 12 and the control line 13 generates a colorization reaction. In this case, the test result is defined as a positive reaction which means that the concentration of the sample (such as ethanol or medicines) in the sample liquid is greater than a predetermined concentration value. At this time, a colorization degree of the test line 12 can be compared with that of the control line 13, in order to evaluate the concentration value of the sample in the sample liquid.

However, referring to Fig. 1C, when the traditionally biological test strip is actually used, the substrate 10 only can provide the single control line 13 for being compared with the color of the test line 12. Thus, when a detection analyzer (not-shown) is used to determine the color darkness, the detection result of the detection analyzer easily includes errors and distortions of concentration values. Furthermore, under a certain condition, the concentration of the sample in the sample liquid may be only slightly greater than the predetermined minimum concentration value, so that the colorization reaction of the test line 12 may be vary weak. At this time, the colorization reaction of the test line 1 is actually a positive reaction, but it may be inaccurately determined to be a negative reaction by naked eyes or the detection analyzer. The foregoing problems of concentration errors or inaccurate determination may substantially lower the operational convenience and the detection accuracy.

Moreover, a traditional method of automatically detecting a test result of a probe zone of a test strip is applied to the field of biological test strip, wherein a scanning object includes a bar code and a plurality of test strips, both of which are arranged on the scanning object in turn. Each of the test strips has a colorization pattern of a control line and a plurality of test lines. The control line and each of the test lines are corresponding to a probe zone on the test strips. After the test strips are reacted with a test liquid, the scanning object are scanned to read the bar code pattern and the images of the test strips. Then, decoding the bar code pattern to obtain the information of types of the test strips, so that a plurality of mapping tables of setting values versus positions of the test lines of one of the test strips can be obtained. Each of the test strips corresponds to one of the mapping tables based on the type of the test strip. Each of the setting values of the mapping table corresponds to one test line of the test strip. Calculating a color response of one test line of the test strip based on the image of the test strip, and comparing the color response of the test line with the setting value from the mapping table corresponding thereto. Thereby, determining whether the test line has a positive test result or a negative test result in response to a specific sample in the test liquid detected by the test strip.

According the foregoing detection method, the bar code of the scanning object can be used to simultaneously detect several detection items of a plurality of different test strips. However, when the scanning object is actually used, each of the test strips still has a single control line for comparing with the color darkness of the test lines on the same test strip. Thus, when a detection analyzer is used to determine the color darkness, the detection result of the detection analyzer still includes errors and distortions of concentration values. Furthermore, if the concentration of the specific sample in the test liquid used by each of the test strips is only slightly greater than a predetermined minimum concentration value, the colorization reaction of the test lines on each of the test strips may be still weak. At this time, there is still a risk that a weak positive reaction may be inaccurately determined to be a negative reaction by the detection analyzer. As a result, the foregoing detection method and the design of the scanning object can not substantially improve the detection accuracy.

As a result, it is important for designers and related manufacturers to think how to develop a biological test strip to solve the problems existing in the traditionally biological test strips, as described above.

### SUMMARY OF THE INVENTION

A primary object of the present invention is to provide a biological test strip, which has at least two control lines for providing at least two color comparison references with different color darkness, so as to correctly compare with and determine the concentration value of at least one test line, for the purpose of efficiently lowering the concentration error and increasing the concentration detection accuracy.

A secondary object of the present invention is to provide a biological test strip, which has at least two control lines for defining a predetermined maximum concentration value and a predetermined minimum concentration value, so as to correctly determine if the colorization reaction of at least one test line is a negative reaction, for the purpose of efficiently lowering the inaccurate determination probability and increasing the operational convenience.

To achieve the above object, the biological test strip of a preferred embodiment of the present invention comprises a source end, at least one test line and at least two control lines. The source end is used for dropping a sample liquid thereon. The at least one test line is used to react with at least one sample of the sample liquid to generate a colorization reaction. The at least two control lines are used to react with the sample liquid to generate at least two colorization reactions with different color darkness, so as to define a predetermined maximum concentration value and a predetermined minimum concentration value, both of which can be used as color comparison references for the at least one test line.

In one embodiment of the present invention, the at least one test line is disposed between the source end and at least one of the control lines.

In one embodiment of the present invention, the at least two control lines comprises a first control line and a second control line, the first control line can generate a colorization reaction to define the predetermined minimum concentration value, while the second control line can generate a colorization reaction to define the predetermined maximum concentration value.

In one embodiment of the present invention, the source end, the first control line, the at least one test line and the second control line are arranged on the biological test strip in turn.

In one embodiment of the present invention, the source end, the second control line, the at least one test line and the first control line are arranged on the biological test strip in turn.

In one embodiment of the present invention, the source end, the at least one test line, the first control line and the second control line are arranged on the biological test strip in turn.

In one embodiment of the present invention, the source end, the at least one test line, the second control line and the first control line are arranged on the biological test strip in turn.

In one embodiment of the present invention, the number of the at least one test line is two or more than two.

In one embodiment of the present invention, the number of the at least two control lines is three or more than three.

In one embodiment of the present invention, the at least two control lines comprises a first control line, a second control line and a third control line, the first control line can generate a colorization reaction to define the predetermined minimum concentration value, the third control line can generate a colorization reaction to define the predetermined maximum concentration value, and the second control line can generate a colorization reaction to define a predetermined intermediate concentration value between the predetermined minimum concentration value and the predetermined maximum concentration value.

### BRIEF DESCRIPTION OF THE DRAWINGS

The structure and the technical means adopted by the present invention to achieve the above and other objects can be best understood by referring to the following detailed description of the preferred embodiments and the accompanying drawings, wherein

Figs. 1A, 1B and 1C are schematic and operational views of a traditionally biological test strip;

Figs. 2A, 2B, 2C and 2D are schematic and operational views of a biological test strip according to a first embodiment of the present invention;

Figs. 3A and 3B are schematic and operational views of a biological test strip according to a second preferred embodiment of the present invention;

Figs. 4A and 4B are schematic and operational views of a biological test strip according to a third preferred embodiment of the present invention;

Figs. 5A and 5B are schematic and operational views of a biological test strip according to a fourth preferred embodiment of the present invention;

Figs. 6A and 6B are schematic and operational views of a biological test strip according to a fifth preferred embodiment of the present invention; and

Figs. 7A and 7B are schematic and operational views of a biological test strip according to a sixth preferred embodiment of the present invention.

### DETAILED DESCRIPTION OF THE PREFERRED EMBODIMENTS

Referring now to Fig. 2A, a biological test strip according to a first embodiment of the present invention is illustrated. As shown, the biological test strip designated by numeral 20 comprises a source end 21, a first control line 22, a test line 23 and a second control line 24, all of which are arranged on the biological test strip 20 in turn. In the present invention, the biological test strip 20 can be made of various test substrates which can guide a liquid to extend, wherein the test substrates can be selected from test paper made of natural cellulose, synthetic cellulose paper (such as nitrocellulose) or other equivalent material. Furthermore, the biological test strip 20 can be installed with a suitable housing or other elements (such as a drying agent, not-shown) to commonly construct a suitable detection device for being conveniently applied to various detections of samples (including ethanol, medicines, cancer molecules or other related disease molecules in the urine or blood), pregnancy, or other biological researches. If a housing is provided, the housing can be labeled with suitable symbols or texts (such as C1, T, C2, and etc.) for conveniently distinguishing the first control line 22, the test line 23 and the second control line 24 from each other. However, as described above, the material, the installation, the application or the label means of the biological test strip 20 can be varied according to actual needs, without limitation.

Referring back to Fig. 2A, in the first embodiment of the present invention, the biological test strip 20 is preferably an elongated paper strip, but the shape is not limited thereto. The shape of the biological test strip 20 can be varied according to the shape of a final product. Furthermore, the source end 21 is formed on a position adjacent to one end of the biological test strip 20. The source end 21 is a zone for dropping a sample liquid thereon. The source end 21 is preferably circular or rectangular, but the shape is not limited thereto. The shape of the source end 21 can be varied according to the shape of the final product.

Referring still to Fig. 2A, in the first embodiment of the present invention, the first control line 22, the test line 23 and the second control line 24 are formed by coating suitable colorization probes on the biological test strip 20 in advance. Meanwhile, the first control line 22, the test line 23 and the second control line 24 can use identical colorization probes with different probe concentrations or use different types of colorization probes. Generally, the colorization probes are colorless before a colorization reaction. After a colorization reaction is finished, the colorization probes can show a predetermined color, such as red or black. The type and color of the colorization probes can be varied according to the application of the biological test strip 20 without limitation. In the first embodiment, the first control line 22 has a colorization probe which can react with the sample liquid to generate a colorization reaction with a relatively lighter color, so as to define a predetermined minimum concentration value used as a color comparison reference for the test line 23. Meanwhile, the second control line 24 has a colorization probe which can react with the sample liquid to generate a colorization reaction with a relatively darker color, so as to define a predetermined maximum concentration value used as a color comparison reference for the test line 23. Furthermore, the test line 23 has a colorization probe which can react with at least one sample in the sample liquid to generate a colorization reaction. The test line 23 is preferably disposed between the source end 21 and the second control line 24. As a result, if the second control line 24 generates a colorization reaction, it can be ensured that the sample liquid has extended and passed through the test line 23 for finishing a detection operation. In one embodiment, the biological test strip 20 is designed based on immuno-chromatographic principle. For example, a region between the source end 21 and the first control line 22 is pre-coated with a specific antigen (or the specific antigen is directly pre-coated on the source end 21). When the sample liquid moves through the region, the antigen will be dissolved into the sample liquid and move with the sample liquid. Thus, the antigen in the sample liquid reacts with a first colorization probe of the first control line 22 and/or the second control line 24 to cause a control colorization reaction. Meanwhile, if the sample liquid further contains a sample defined as an antibody, the antibody in the sample liquid reacts with a second colorization probe of the test line 23 to cause a test colorization reaction. On the other hand, if the sample liquid contains no sample, there will be no test colorization reaction between the sample liquid and the test line 23, while only the antigen in the sample liquid reacts with the first control line 22 and the second control line 24 to cause the control colorization reaction. However, the present invention also can be carried out by other equivalent colorization principle without limitation.

Referring now to Figs. 2B, 2C and 2D, when the biological test strip 20 of the first embodiment of the present invention is used to detect if a sample liquid contains a sample (such as ethanol or medicine), the sample liquid is firstly dropped to the source end 21. After this, the sample liquid (and the sample therein) extends toward and passes through the first control line 22, the test line 23 and the second control line 24 in turn. As shown in Fig. 2B, after the sample liquid is dropped for a test, the first control line 22 generates a colorization reaction with a relatively lighter color, the second control line 24 generates a colorization reaction with a relatively darker color, and the test line 23 is still colorless (or has a color weaker than that of the first control line 22). In this case, the test result will be a negative reaction. According to the present invention, a detection analyzer (not-shown) is used to execute a quantitative analysis, wherein the detection analyzer uses color comparison references defined by the predetermined minimum concentration value of the first control line 22 and the predetermined maximum concentration value of the second control line 24. Thus, the color darkness of the test line 23 can be calculated and evaluated by an algorithm (such as an interpolation algorithm), so as to speedily obtain the concentration of the sample in the sample liquid. As shown in Fig. 2B, the detection result shows that the concentration of the sample (such as ethanol or medicine) in the sample liquid is zero or less than the predetermined minimum concentration value.

Referring now to Fig. 2C, after the sample liquid is dropped for a test, the detection analyzer similarly uses the color comparison references defined by the predetermined minimum concentration value of the first control line 22 and the predetermined maximum concentration value of the second control line 24, and then calculates the color darkness generated by a colorization reaction of the test line 23 based on the interpolation algorithm. If the color darkness of the test line 23 is sandwiched between that of the first control line 22 and the second control line 24, the test result will be a positive reaction, wherein the concentration of the sample in the sample liquid is greater than the predetermined minimum concentration value but less than the predetermined maximum concentration value.

Referring now to Fig. 2D, after the sample liquid is dropped for a test, the detection analyzer similarly uses the color comparison references defined by the predetermined minimum concentration value of the first control line 22 and the predetermined maximum concentration value of the second control line 24, and then calculates the color darkness generated by a colorization reaction of the test line 23 based on the interpolation algorithm. If the color darkness of the test line 23 is greater than that of the first control line 22 and the second control line 24, the test result will be an overdose positive reaction, wherein the concentration of the sample in the sample liquid is greater than the predetermined maximum concentration value.

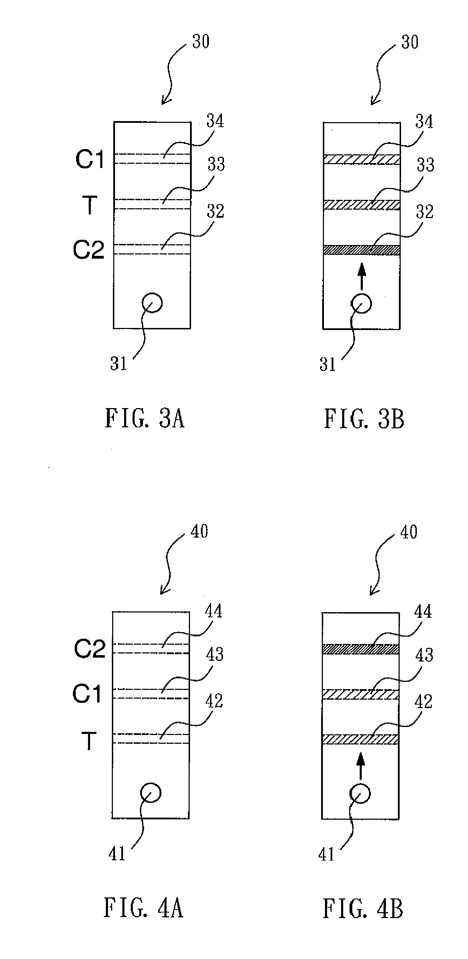

Referring now to Figs. 3A and 3B, a biological test strip according to a second embodiment of the present invention is illustrated and similar to the first embodiment. As shown, the biological test strip designated by numeral 30 comprises a source end 31, a second control line 32, a test line 33 and a first control line 34, all of which are arranged on the biological test strip 30 in turn, wherein the arrangement order of the second embodiment is different from that of the first embodiment. For conveniently distinguishing these lines from each other, Figs. 3A and 3B are additionally labeled by "C2", "T" and "C1" to represent the second control line 32, the test line 33 and the first control line 34 in turn. In the second embodiment, the first control line 34 has a colorization probe which can react with the sample liquid to generate a colorization reaction with a relatively lighter color, so as to define a predetermined minimum concentration value used as a color comparison reference for the test line 33. Meanwhile, the second control line 32 has a colorization probe which can react with the sample liquid to generate a colorization reaction with a relatively darker color, so as to define a predetermined maximum concentration value used as a color comparison reference for the test line 33. Thus, as shown in Fig. 3B, after the sample liquid is dropped for a test, the detection analyzer uses the color comparison references defined by the predetermined minimum concentration value of the first control line 34 and the predetermined maximum concentration value of the second control line 32, and then calculates the color darkness generated by a colorization reaction of the test line 33 based on the interpolation algorithm. If the color darkness of the test line 33 is sandwiched between that of the first control line 34 and the second control line 32, the test result will be a positive reaction, wherein the concentration of the sample in the sample liquid is greater than the predetermined minimum concentration value but less than the predetermined maximum concentration value. Furthermore, in the second embodiment, the principles of a negative reaction or an overdose positive reaction are similar to that of the first embodiment, so that the detailed description thereof will be omitted hereinafter.

Referring now to Figs. 4A and 4B, a biological test strip according to a third embodiment of the present invention is illustrated and similar to the first and second embodiments. As shown, the biological test strip designated by numeral 40 comprises a source end 41, a test line 42, a first control line 43 and a second control line 44, all of which are arranged on the biological test strip 40 in turn, wherein the arrangement order of the third embodiment is different from that of the first and second embodiments. For conveniently distinguishing these lines from each other, Figs. 4A and 4B are additionally labeled by "T", "C1" and "C2" to represent the test line 42, the first control line 43 and the second control line 44 in turn. In the third embodiment, the first control line 43 has a colorization probe which can react with the sample liquid to generate a colorization reaction with a relatively lighter color, so as to define a predetermined minimum concentration value used as a color comparison reference for the test line 42. Meanwhile, the second control line 44 has a colorization probe which can react with the sample liquid to generate a colorization reaction with a relatively darker color, so as to define a predetermined maximum concentration value used as a color comparison reference for the test line 42. Thus, as shown in Fig. 4B, after the sample liquid is dropped for a test, the test line 42 generates a colorization reaction. If the color darkness of the colorization reaction of the test line 42 is determined to be sandwiched between that of the first control line 43 and the second control line 44, the test result will be a positive reaction, wherein the concentration of the sample in the sample liquid is greater than the predetermined minimum concentration value but less than the predetermined maximum concentration value. Furthermore, in the third embodiment, the principles of a negative reaction or an overdose positive reaction are similar to that of the first and second embodiments, so that the detailed description thereof will be omitted hereinafter.

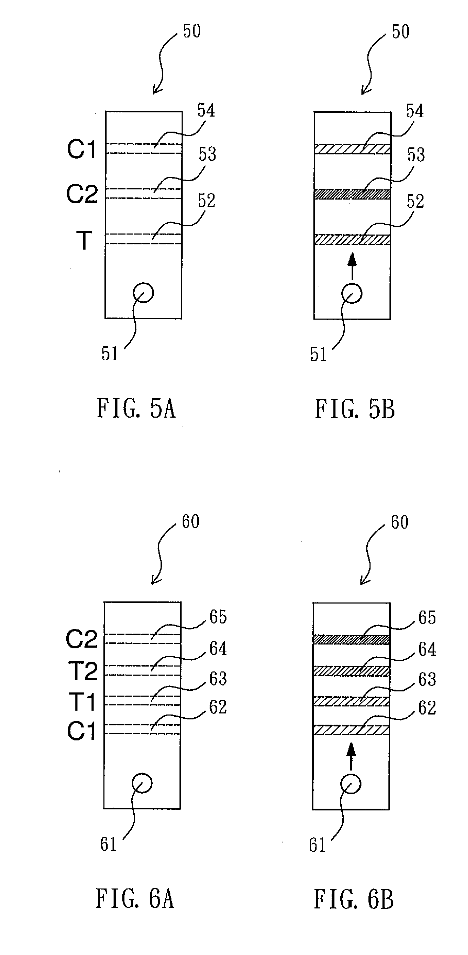

Referring now to Figs. 5A and 5B, a biological test strip according to a fourth embodiment of the present invention is illustrated and similar to the first, second and third embodiments. As shown, the biological test strip designated by numeral 50 comprises a source end 51, a test line 52, a second control line 53 and a first control line 54, all of which are arranged on the biological test strip 50 in turn, wherein the arrangement order of the fourth embodiment is different from that of the first, second and third embodiments. For conveniently distinguishing these lines from each other, Figs. 5A and 5B are additionally labeled by "T", "C2" and "C1" to represent the test line 52, the second control line 53 and the first control line 54 in turn. In the fourth embodiment, the first control line 54 has a colorization probe which can react with the sample liquid to generate a colorization reaction with a relatively lighter color, so as to define a predetermined minimum concentration value used as a color comparison reference for the test line 52. Meanwhile, the second control line 53 has a colorization probe which can react with the sample liquid to generate a colorization reaction with a relatively darker color, so as to define a predetermined maximum concentration value used as a color comparison reference for the test line 52. Thus, as shown in Fig. 5B, after the sample liquid is dropped for a test, the test line 52 generates a colorization reaction. If the color darkness of the colorization reaction of the test line 52 is determined to be sandwiched between that of the first control line 54 and the second control line 53, the test result will be a positive reaction, wherein the concentration of the sample in the sample liquid is greater than the predetermined minimum concentration value but less than the predetermined maximum concentration value. Furthermore, in the fourth embodiment, the principles of a negative reaction or an overdose positive reaction are similar to that of the first, second and third embodiments, so that the detailed description thereof will be omitted hereinafter.

Referring now to Figs. 6A and 6B, a biological test strip according to a fifth embodiment of the present invention is illustrated and similar to the first, second, third and fourth embodiments. As shown, the biological test strip designated by numeral 60 comprises a source end 61, a first control line 62, a first test line 63, a second test line 64 and a second control line 65, all of which are arranged on the biological test strip 60 in turn, wherein the number of the test lines 63, 64 can be two or more than two, while the arrangement order of the test lines 63, 64, the first control line 62 and the second control line 65 can be varied according to that of the first, second, third or fourth embodiments. For conveniently distinguishing these lines from each other, Figs. 6A and 6B are additionally labeled by "C1", "T1", "T2" and "C2" to represent the first control line 62, the first test line 63, the second test line 64 and the second control line 65 in turn. In the fifth embodiment, the first control line 62 has a colorization probe which can react with the sample liquid to generate a colorization reaction with a relatively lighter color, so as to define a predetermined minimum concentration value used as a color comparison reference for the test lines 63, 64. Meanwhile, the second control line 65 has a colorization probe which can react with the sample liquid to generate a colorization reaction with a relatively darker color, so as to define a predetermined maximum concentration value used as a color comparison reference for the test lines 63, 64. Thus, as shown in Fig. 6B, after the sample liquid is dropped for a test, each of the test lines 63, 64 generates a colorization reaction. If the color darkness of the colorization reaction of the test line 63 or 64 is determined to be sandwiched between that of the first control line 62 and the second control line 65, the test result will be a positive reaction, wherein the concentration of the sample in the sample liquid is greater than the predetermined minimum concentration value but less than the predetermined maximum concentration value. Furthermore, in the fifth embodiment, the principles of a negative reaction or an overdose positive reaction are similar to that of the first, second, third and fourth embodiments, so that the detailed description thereof will be omitted hereinafter.

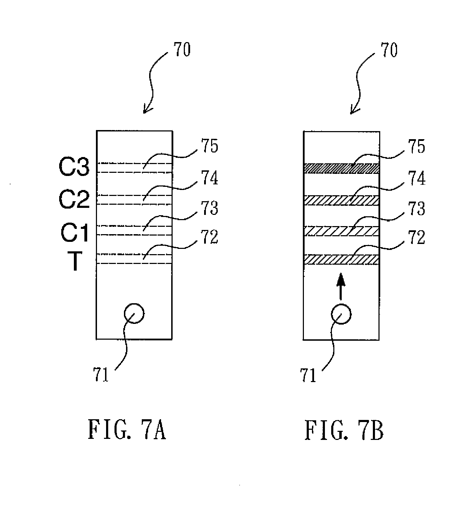

Referring now to Figs. 7A and 7B, a biological test strip according to a sixth embodiment of the present invention is illustrated and similar to the first, second, third, fourth and fifth embodiments. As shown, the biological test strip designated by numeral 70 comprises a source end 71, a test line 72, a first control line 73, a second control line 74 and a third control line 75, all of which are arranged on the biological test strip 70 in turn, wherein the number of the control lines 73, 74, 75 can be three or more than three, while the arrangement order of the test line 72, the first control line 73, the second control line 74 and the third control line 75 can be varied according to that of the first, second, third or fourth embodiments. For conveniently distinguishing these lines from each other, Figs. 7A and 7B are additionally labeled by "T", "C1", "C2" and "C3" to represent the test line 72, the first control line 73, the second control line 74 and the third control line 75 in turn. In the sixth embodiment, the first control line 73 has a colorization probe which can react with the sample liquid to generate a colorization reaction with a relatively lighter color, so as to define a predetermined minimum concentration value used as a color comparison reference for the test line 72. Meanwhile, the third control line 75 has a colorization probe which can react with the sample liquid to generate a colorization reaction with a relatively darker color, so as to define a predetermined maximum concentration value used as a color comparison reference for the test line 72. The second control line 74 can generate a colorization reaction to define a predetermined intermediate concentration value between the predetermined minimum concentration value and the predetermined maximum concentration value, wherein the predetermined intermediate concentration value is also used as a color comparison reference for the test line 72. Thus, as shown in Fig. 7B, after the sample liquid is dropped for a test, the test line 72 generates a colorization reaction. If the color darkness of the colorization reaction of the test line 72 is determined to be sandwiched between that of the first control line 73 and the second control line 74 (or between that of the second control line 74 and the third control line 75), the test result will be a positive reaction, wherein the concentration of the sample in the sample liquid is greater than the predetermined minimum concentration value but less than the predetermined maximum concentration value. As a result, the concentration of the sample can be detected more definitely. Furthermore, in the sixth embodiment, the principles of a negative reaction or an overdose positive reaction are similar to that of the first, second, third, fourth and fifth embodiments, so that the detailed description thereof will be omitted hereinafter.

As described above, in comparison with the traditionally biological test strip which only has the single control line 13 for comparing with the color darkness of the test line 12 and may cause the concentration errors or inaccurate determination when the detected result is detected by the detection analyzer, the biological test strip 20 of the present invention, as shown in Figs. 2A to 2D, has the at least two control lines 22, 24 for providing at least two color comparison references with different color darkness (i.e. for defining a predetermined maximum concentration value and a predetermined minimum concentration value), so as to correctly compare with the concentration value of the at least one test line 23 and correctly determine if the colorization reaction of the test line 23 is a negative reaction. As a result, the concentration error and the inaccurate determination probability can be efficiently lowered, while the concentration detection accuracy and the operational convenience can be efficiently increased.

The present invention has been described with a preferred embodiment thereof and it is understood that many changes and modifications to the described embodiment can be carried out without departing from the scope and the spirit of the invention that is intended to be limited only by the appended claims.

## Claims

1. A biological test strip, comprising:
a source end for dropping a sample liquid thereon;
at least one test line for reacting with at least one sample of the sample liquid to generate a colorization reaction; and
at least two control lines for reacting with the sample liquid to generate at least two colorization reactions with different color darkness, so as to define a predetermined maximum concentration value and a predetermined minimum concentration value, both of which are used as color comparison references for the at least one test line.

2. The biological test strip of claim 1, wherein the at least one test line is disposed between the source end and at least one of the control lines.

3. The biological test strip of claim 1, wherein the at least two control lines comprises a first control line and a second control line, the first control line can generate a colorization reaction to define the predetermined minimum concentration value, while the second control line can generate a colorization reaction to define the predetermined maximum concentration value.

4. The biological test strip of claim 3, wherein the source end, the first control line, the at least one test line and the second control line are arranged on the biological test strip in turn.

5. The biological test strip of claim 4, wherein the number of the at least one test line is two or more than two.

6. The biological test strip of claim 3, wherein the source end, the second control line, the at least one test line and the first control line are arranged on the biological test strip in turn.

7. The biological test strip of claim 6, wherein the number of the at least one test line is two or more than two.

8. The biological test strip of claim 3, wherein the source end, the at least one test line, the first control line and the second control line are arranged on the biological test strip in turn.

9. The biological test strip of claim 8, wherein the number of the at least one test line is two or more than two.

10. The biological test strip of claim 3, wherein the source end, the at least one test line, the second control line and the first control line are arranged on the biological test strip in turn.

11. The biological test strip of claim 10, wherein the number of the at least one test line is two or more than two.

12. The biological test strip of claim 1, wherein the number of the at least two control lines is three or more than three.

13. The biological test strip of claim 12, wherein the at least two control lines comprises a first control line, a second control line and a third control line, the first control line can generate a colorization reaction to define the predetermined minimum concentration value, the third control line can generate a colorization reaction to define the predetermined maximum concentration value, and the second control line can generate a colorization reaction to define a predetermined intermediate concentration value between the predetermined minimum concentration value and the predetermined maximum concentration value.

14. The biological test strip of claim 13, wherein the number of the at least one test line is two or more than two.
